# EUROPÄISCHE PATENTSCHRIFT

(11) **EP 1 902 005 B1**
(45) Veröffentlichungstag und Bekanntmachung des Hinweises auf die Patenterteilung: **08.08.2012**
(21) Anmeldenummer: 06776076.9
(22) Anmeldetag: 23.06.2006
(51) Int. Cl.: C07C 1/20, C07C 11/06, C07C 4/06

(54) **VERFAHREN UND ANLAGE ZUR HERSTELLUNG VON C2-C4- OLEFINEN AUS METHANOL UND/ODER DIMETHYLETHER MIT ERHÖHTER AUSBEUTE**
METHOD AND SYSTEM FOR PRODUCING INCREASED YIELDS OF C2-C4 OLEFINS FROM METHANOL AND/OR DIMETHYL ETHER
PROCEDE ET INSTALLATION POUR PRODUIRE DES OLEFINES C2-C4 A PARTIR DE METHANOL ET/OU DE DIMETHYLETHER AVEC DES RENDEMENTS ACCRUS

(30) Priorität: 24.06.2005 DE 102005029399
(43) Veröffentlichungstag der Anmeldung: 26.03.2008
(73) Patentinhaber: Lurgi AG, 60295 Frankfurt am Main (DE)
(72) Erfinder: ROTHAEMEL, Martin, 58452 Witten (DE); FINCK, Uwe, 6699 Kjorsvikbugen (NO); RENNER, Thomas, 60318 Frankfurt (DE); BUCHOLD, Henning, 63452 Hanau (DE)
(74) Vertreter: KEIL & SCHAAFHAUSEN Patentanwälte
(86) Internationale Anmeldenummer: PCT/EP2006/006065
(87) Internationale Veröffentlichungsnummer: WO 2006/136433

(56) Entgegenhaltungen:
- EP-A- 0 145 234
- EP-A- 0 882 692

## Beschreibung

Die vorliegende Erfindung betrifft ein Verfahren zur Herstellung von C₂-C₄-Olefinen, insbesondere von Propylen aus einem Wasserdampf sowie Methanol- und/oder Dimethyletherdampf enthaltenden Eduktgemisch, bei dem das Eduktgemisch in wenigstens einem ersten Reaktor an einem körnigen, formselektiven Zeolith-Katalysator zu einem niedermolekulare Olefine und Benzinkohlenwasserstoffe umfassenden Reaktionsgemisch umgesetzt wird, welches in einer ersten Trenneinrichtung in ein C₂-C₄-olefinreiches Gemisch, ein an C₅₊-Benzinkohlenwasserstoffen reiches Gemisch und eine wässrige Phase aufgetrennt wird, wobei das benzinkohlenwasserstoffreiche Gemisch mit einem inerten Medium vermischt, die so erhaltene Mischung in wenigstens einem zweiten Reaktor an körnigem Zeolith-Katalysator zu einem C₂-C₄-Olefine umfassenden Produktgemisch umgesetzt und dieses Produktgemisch zu der ersten Trenneinrichtung zurückgeführt wird. Des weiteren betrifft die vorliegende Erfindung eine zur Durchführung des Verfahrens geeignete Anlage.

Zur Herstellung von niedermolekularen C₂-C₄-Olefinen, insbesondere Propylen, aus Methanol- und/oder Dimethylether sind dem Fachmann eine Vielzahl an Verfahren bekannt, welche üblicherweise auf der Umsetzung eines Wasserdampf sowie Methanol- und/oder Dimethyletherdampf enthaltenden Eduktgemischs an einem formselektiven Zeolith-Katalysator basieren.

In der DE 100 27 159 A1 wird ein Verfahren zur Herstellung von Propylen aus Methanol offenbart, bei dem zunächst aus Methanoldampf an einem ersten Katalysator ein Dimethylether enthaltendes Dampfgemisch erzeugt wird, bevor dieses mit Wasserdampf vermischt und in wenigstens zwei in Serie geschalteten Schachtreaktoren mit Katalysatorschüttungen aus formselektivem Zeolith zu einem propylenhaltigen Produktgemisch umgesetzt wird. Anschließend wird das Produktgemisch in einer mehrere Destillationskolonnen umfassenden Trenneinrichtung aufgearbeitet, wobei eine propylenreiche Fraktion mit einem Propylengehalt von wenigstens 95 Vol.-%, eine niedermolekulare Kohlenwasserstoffe enthaltende Fraktion, welche in die Katalysatorschüttungen zurückgeführt wird, und eine benzinkohlenwasserstoffreiche Fraktion, welche aus dem Verfahren entfernt wird, erhalten wird. Nachteilig an diesem Verfahren ist jedoch die, bezogen auf den Gesamtkohlenstoffgehalt in dem Eduktgemisch, geringe Ausbeute an Propylen, welche unter anderem darin begründet liegt, dass die benzinkohlenwasserstoffreiche Fraktion ungenutzt aus dem Verfahren entfernt wird.

Aus der EP 0 882 692 B1 ist ein Verfahren zur Herstellung von C₂-C₃-Olefinen bekannt, bei dem ein Gemisch aus Wasserdampf und Methanol- und/oder Dimethyletherdampf in einem einen Zeolith-Katalysator enthaltenden Röhrenreaktor bei einer Temperatur zwischen 280 und 570°C und einem Druck zwischen 0,1 und 0,9 bar zu einem olefinreichen Produktgemisch umgesetzt wird, welches anschließend in einer Trenneinrichtung in eine C₂-C₄-Olefinfraktion mit einem Propylengehalt von wenigstens 40 Gew.-%, eine wasserhaltige Fraktion, eine gasförmige Fraktion und eine C₅₊-Benzinkohlenwasserstoffe enthaltende Fraktion aufgetrennt wird. Während die drei erstgenannten Fraktionen aus dem Verfahren abgezogen werden, wird der die C₅₊-Benzinkohlenwasserstoffe enthaltende Produktstrom mit Wasser vermischt, in einem Erhitzer auf eine Temperatur von 380 bis 700°C erhitzt und in einem zweiten, einen Zeolith-Katalysator enthaltenden Reaktor zu C₂-C₄-Olefinen umgesetzt, bevor die Reaktionsprodukte zu der Trenneinrichtung zurückgeführt werden. Die mit diesem Verfahren erhaltenen Ausbeuten an C₂-C₃-Olefinen sind, obgleich höher als die mit dem aus der DE 100 27 159 A1 bekannten Verfahren, bei dem die benzinkohlenwasserstoffreiche Fraktion ungenutzt aus dem Verfahren entfernt wird, erhaltenen, ebenfalls verbesserungsbedürftig. Zudem zeichnet sich dieses Verfahren nicht zuletzt wegen der isothermen Verfahrensführung sowie dem notwendigen Vakuumbetrieb in dem Röhrenreaktor durch hohe Kosten aus.

Aufgabe der vorliegenden Erfindung ist die Bereitstellung eines Verfahrens zur Herstellung von C₂-C₄-Olefinen, insbesondere von Propylen, aus einem Wasserdampf sowie Methanol- und/oder Dimethyletherdampf enthaltenden Eduktgemisch mit möglichst hoher Ausbeute.

Erfindungsgemäß wird diese Aufgabe durch ein Verfahren mit den Merkmalen des Patentanspruchs 1 gelöst.

Überraschenderweise konnte im Rahmen der vorliegenden Erfindung herausgefunden werden, dass die Ausbeute an C₂-C₄-Olefinen bei einem Verfahren zur Herstellung von C₂-C₄-Olefinen, insbesondere von Propylen, aus einem Wasserdampf sowie Methanol- und/oder Dimethyletherdampf enthaltenden Eduktgemisch ohne Erhöhung der Betriebskosten signifikant gesteigert wird, wenn die aus dem nach der Umsetzung des Eduktgemischs in dem ersten Reaktor erhaltenen Reaktionsgemisch in der ersten Trenneinrichtung abgetrennte, C₅₊-Benzinkohlenwasserstoffe enthaltende Fraktion zunächst in einer zweiten Trenneinrichtung in einen C₅-C₆-Kohlenwasserstoffe umfassenden Produktstrom sowie einen C₇₊-Kohlenwasserstoffe umfassenden Produktstrom aufgetrennt wird und nur der die C₇₊-Kohlenwasserstoffe enthaltende Produktstrom in dem zweiten Reaktor umgesetzt wird, wohingegen der C₅-C₆-Kohlenwasserstoffe enthaltende Produktstrom zusammen mit dem Eduktgemisch dem wenigstens einen ersten Reaktor zugeführt und dort erneut zu niedermolekularen Olefinen umgesetzt wird. Sowohl der C₅-C₆-Kohlenwasserstoffe enthaltende Produktstrom als auch der C₇₊-Kohlenwasserstoffe enthaltende Produktstrom enthält nach den Erkenntnissen der vorliegenden Erfindung unerwartet hohe Mengen an an einem formselektiven Zeolith-Katalysator zu C₂-C₄-Olefinen umsetzbaren Verbindungen. Während die zweitgenannte Fraktion signifikante Mengen an Aromaten, welche mit Methanol und/oder Dimethylether unter Bildung von Alkylaromaten reagieren, aufweist und daher nicht zusammen mit dem methanol- und/oder dimethyletherhaltigen Eduktgemisch umgesetzt werden kann, ist die erstgenannte Fraktion im Wesentlichen frei an Aromaten und kann daher ohne Gefahr von unerwünschten Nebenreaktionen in dem ersten Reaktor eingesetzt werden.

Vorzugsweise wird aus dem an C₅₊-Benzinkohlenwasserstoffen reichen Gemisch in der zweiten Trenneinrichtung ein C₇₊-Kohlenwasserstoffe enthaltender Produktstrom abgetrennt, welcher 10 bis 30 Gew.-% C₇₊-Paraffine, 40 bis 70 Gew.-% Aromaten, 5 bis 20 Gew.-% Naphthene, 5 bis 25 Gew.-% C₇₊-Olefine sowie weniger als 20 Gew.-% C₆₋-Kohlenwasserstoffe umfasst, da diese Zusammensetzungen einen hohen Anteil an an einem Zeolith-Katalysator zu C₂-C₄-Olefinen und insbesondere zu Propylen umsetzbaren Verbindungen enthalten. Besonders gute Ergebnisse werden erhalten, wenn der in der zweiten Trenneinrichtung abgetrennte C₇₊-Produktstrom 15 bis 25 Gew.-% C₇₊-Paraffine, 40 bis 50 Gew.-% Aromaten, 15 bis 20 Gew.-% Naphthene, 15 bis 25 Gew.-% C₇₊-Olefine sowie weniger als 10 Gew.-% C₆₋-Kohlenwasserstoffe enthält. Ganz besonders bevorzugt beträgt der Anteil an C₆₋-Kohlenwasserstoffen, also an Kohlenwasserstoffen mit einer Kettenlänge von 6 oder weniger C-Atomen, in dem C₇₊-Produktstrom weniger als 5 Gew.-%.

In Weiterbildung des Erfindungsgedankens wird vorgeschlagen, in der zweiten Trenneinrichtung einen C₅-C₆-Kohlenwasserstoffe enthaltenden Produktstrom abzutrennen, welcher weniger als 5 Gew.-% Aromaten, bevorzugt weniger als 2,5 Gew.-% Aromaten und besonders bevorzugt weniger als 1 Gew.-% Aromaten enthält. Auch diese Zusammensetzungen weisen hohe Anteile an an einem Zeolith-Katalysator zu C₂-C₄-Olefinen und insbesondere zu Propylen umsetzbaren Verbindungen auf. Zudem wird durch den niedrigen Aromatenanteil sichergestellt, dass bei der nachfolgenden Reaktion des C₅-C₆-Produktstroms in dem ersten Reaktor keine unerwünschten Nebenreaktionen, insbesondere keine Alkylierungsreaktionen mit Methanol, stattfinden.

Grundsätzlich kann die zweite Trenneinrichtung in jeder dem Fachmann bekannten Weise ausgebildet sein, sofern sie daran angepasst ist, ein C₅₊-Benzinkohlenwasserstoff reiches Gemisch in einen C₅-C₆-Kohlenwasserstoffe enthaltenden und einen C₇₊-Kohlenwasserstoffe enthaltenden Produktstrom aufzutrennen. Lediglich beispielsweise seien destillativ, adsorptiv, thermisch oder mittels Membranen arbeitende Trenneinrichtungen genannt, wobei insbesondere mit Destillationskolonnen gute Ergebnisse erhalten werden.

Als dem in der zweiten Trenneinrichtung gebildeten C₇₊-Kohlenwasserstoff enthaltenden Produktstrom vor dem zweiten Reaktor zuzuführendes inertes Medium haben sich insbesondere Wasser, Stickstoff und/oder Butan bewährt, wobei Wasser für diesen Zweck besonders bevorzugt ist.

Erfindungsgemäß kann das dem ersten Reaktor zugeführte Eduktgemisch neben Wasserdampf, bezogen auf dessen wasserdampffreien Anteil, 0 bis 100 Gew.-% Methanol und 100 bis 0 Gew.-% Dimethylether enthalten. Methanol- und dimethyletherhaltige Gasgemische können bspw. in an sich bekannter Weise durch partielle Umwandlung von Methanol an einem körnigen Aluminiumoxidkatalysator erzeugt werden, wobei die Reaktion vorzugsweise bei einer Temperatur zwischen 200 und 350°C durchgeführt wird.

Vorzugsweise ist der erste Reaktor als Schachtreaktor, Röhrenreaktor, stationärer Wirbelschichtreaktor oder zirkulierender Wirbelschichtreaktor ausgebildet. Im zweitgenannten Fall sind in dem Reaktor vorzugsweise mehrere axial angeordnete Röhren vorgesehen, welche bspw. eine Länge zwischen 1 und 5 Meter sowie einen inneren Durchmesser von 20 bis 50 mm aufweisen.

Um eine möglichst hohe Umsetzung des Eduktgemischs zu erreichen, wird dieses gemäß einer besonderen Ausführungsform der vorliegenden Erfindung durch zwei oder mehr hintereinander geschaltete erste Reaktoren geführt. Für diese Ausführungsform haben sich insbesondere mehr als zwei, bevorzugt drei, in Serie geschaltete Schachtreaktoren jeweils mit einem formselektiven Zeolith-Katalysator als besonders geeignet erwiesen, wobei in den ersten Schachtreaktor ein Teil des Eduktgemischs aus dem Vorreaktor und in jeden weiteren Schachtreaktor das Produktgemisch des diesem jeweils vorgeschalteten Schachtreaktors zusammen mit einem Teilstrom des Eduktgemischs aus dem Vorreaktor geleitet werden.

Ebenso gute Umsetzungsgrade werden erhalten, wenn alternativ zu der vorgenannten Ausführungsform das Eduktgemisch durch nur einen ersten Reaktor geführt wird, in dem wenigstens zwei hintereinander angeordnete Katalysatorstufen vorgesehen sind. In diesem Fall sind die einzelnen Katalysatorstufen vorzugsweise untereinander angeordnet und werden von dem Eduktgemisch von oben nach unten durchströmt. Auch hier wird das Eduktgemisch aus dem Vorreaktor auf die einzelnen Katalysatorstufen verteilt.

Grundsätzlich können in dem wenigstens einen ersten Reaktor alle dem Fachmann zur Umsetzung von Methanol und/oder Dimethylether zu C₂-C₄-Olefinen geeignete Zeolith-Katalysatoren eingesetzt werden, wobei sich insbesondere Alumosilikat-Zeolith vom Pentasiltyp und besonders bevorzugt ZSM-5 als besonders geeignet erwiesen haben. Zur Optimierung der Ausbeute ist es weiterhin bevorzugt, dem Reaktor, d.h. dem ersten Reaktor bzw. der ersten Reaktorstufe, mindestens einen inerten Strom, besonders bevorzugt Wasserdampf, und mindestens einen Kohlenwasserstoffe enthaltenden Strom zuzuführen.

Um die Betriebskosten des Verfahrens abzusenken, wird in Weiterbildung des Erfindungsgedankens vorgeschlagen, die Umsetzung in dem wenigstens einen ersten Reaktor und/oder in dem wenigstens einen zweiten Reaktor adiabatisch durchzuführen. Alternativ dazu ist in den vorgenannten Reaktoren, wenn auch weniger bevorzugt, eine isotherme Verfahrensführung, welche verglichen mit der adiabatischen Verfahrensführung allerdings zu höheren Verfahrenskosten führt, möglich.

Gute Ausbeuten in dem wenigstens einen ersten Reaktor werden insbesondere erhalten, wenn diesem ein Eduktgemisch mit einem Gewichtsverhältnis Wasser zu Methanoläquivalent von 0,25:1 bis 6:1 zugeführt wird. Sofern der Reaktor mehrere Katalysatorstufen umfasst, gilt dieses Verhältnis für den Eintritt in jede Katalysatorstufe. Ein "Methanoläquivalent" entspricht nach der Gleichung 2 CH₃OH → CH₃-O-CH₃+H₂O einem halben Mol Dimethylether (DME). Zudem wird das Eduktgemisch in dem ersten Reaktor bevorzugt bei einer Temperatur von 300 bis 600°C und/oder bei einem Druck von 0,5 und 5 bara umgesetzt.

Zur Auftrennung des aus dem ersten Reaktor abgezogenen Reaktionsgemischs kann jede dem Fachmann bekannte Trenneinrichtung eingesetzt werden, die geeignet ist, ein C₂-C₄-olefinreiches Gemisch von einem an C₅₊-Benzinkohlenwasserstoffen reichen Gemisch abzutrennen, beispielsweise destillativ, adsorptiv, thermisch oder mittels Membranen arbeitende Trenneinrichtungen. Besonders gute Ergebnisse werden erhalten, wenn die erste Trenneinrichtung als Kühlvorrichtung ausgebildet ist und das aus dem ersten Reaktor abgezogene Reaktionsgemisch in dieser auf eine Temperatur von 10 bis 80°C abgekühlt wird.

Gemäß einer weiteren bevorzugten Ausführungsform der vorliegenden Erfindung wird das aus der ersten Trenneinrichtung abgezogene C₂-C₄-Olefinreiche Gemisch einer dritten Trenneinrichtung zugeführt, in der das vorgenannte Gemisch in einen C₄-C₅-Olefin- und einen C₃₋-Olefinstrom aufgetrennt wird. Dies ermöglicht die Rückführung des C₄-C₅-Olefinstrom in den ersten Reaktor, womit die Gesamtausbeute des Verfahrens weiter gesteigert werden kann. Aus dem C₃₋-Olefinstrom kann Propylen bspw. destillativ in einfacher Weise und mit hoher Reinheit gewonnen werden. Vorzugsweise werden auch die nach dem Abtrennen von Propylen aus dem C₃₋-Olefinstrom verbleibenden Olefine in den ersten Reaktor zurückgeführt.

In dem zweiten Reaktor können grundsätzlich dieselben Katalysatoren wie in dem ersten Reaktor eingesetzt werden, wobei Alumosilikat-Zeolith vom Pentasiltyp und insbesondere ZSM-5 bevorzugt sind. Gute Ausbeuten werden in dem zweiten Reaktor insbesondere erhalten, wenn diesem eine Mischung mit einem Verhältnis von Wasser zu Kohlenwasserstoff von 0,1:1 bis 3:1 und bevorzugt von mindestens 1:1 zugeführt wird. Zudem wird der Produktstrom in dem zweiten Reaktor vorzugsweise bei einer Temperatur von 380 bis 700°C und besonders bevorzugt von 400 bis 600°C und/oder vorzugsweise bei einem Druck von 0,2 und 5 bara und besonders bevorzugt von 1,0 bis 2,5 bara umgesetzt.

Ein weiterer Gegenstand der vorliegenden Erfindung ist eine Anlage zur Herstellung von C₂-C₄-Olefinen, insbesondere von Propylen, aus einem Wasserdampf sowie Methanol- und/oder Dimethyletherdampf enthaltenden Eduktgemisch, welche insbesondere zur Durchführung des erfindungsgemäßen Verfahrens geeignet ist. Erfindungsgemäß umfasst die Anlage wenigstens einen ersten Reaktor und wenigstens einen zweiten Reaktor, wobei beide Reaktoren jeweils einen körnigen, formselektiven Zeolith-Katalysator enthalten, sowie eine erste Trenneinrichtung zur Auftrennung des in dem ersten Reaktor erhaltenen Reaktionsgemischs, wobei in der Anlage des weiteren eine zweite Trenneinrichtung vorgesehen ist, weiche daran angepasst ist, ein an C₅₊-Benzinkohlenwasserstoffen reiches Gemisch vor der Zuführung in den zweiten Reaktor in einen C₅-C₆-Kohlenwasserstoffe enthaltenden und einen C₇₊-Kohlenwasserstoffe enthaltenden Produktstrom aufzutrennen, und wobei von dem zweiten Reaktor eine Rückfuhrleitung zu der ersten Trenneinrichtung führt.

Gemäß einer bevorzugten Ausführungsform der vorliegenden Erfindung ist der ersten Trenneinrichtung eine dritte Trenneinrichtung zur Auftrennung des aus der ersten Trenneinrichtung abgezogenen C₂-C₄-olefinreichen Gemischs in einen C₄-C₅-Olefin- und einen C₃₋-Olefinstrom nachgeschaltet, wobei die dritte Trenneinrichtung zumindest eine Destillationskolonne umfasst.

In Weiterbildung des Erfindungsgedankens wird vorgeschlagen, in der Anlage eine von der dritten Trenneinrichtung zu dem ersten Reaktor führende Rückführleitung vorzusehen, um das aus der dritten Trenneinrichtung abgezogene C₄-C₅-olefinreiche Gemisch in den ersten Reaktor zurückzuführen.

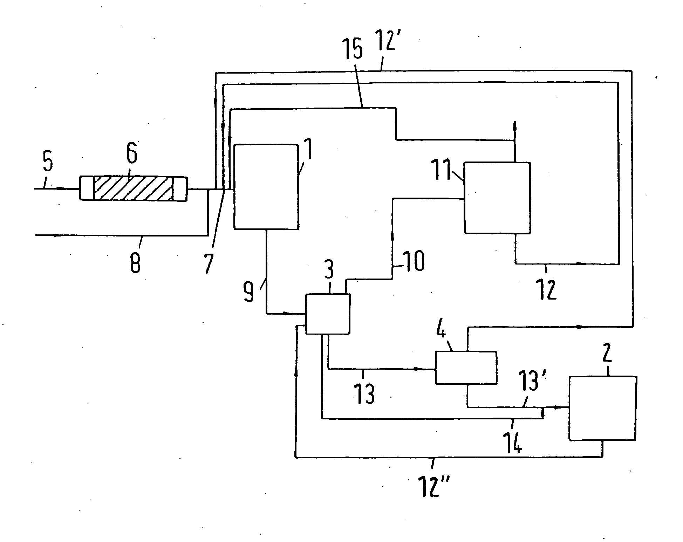

Weitere Merkmale, Vorteile und Anwendungsmöglichkeiten der Erfindung ergeben sich aus der nachfolgenden Beschreibung von Ausführungsbeispielen und der Zeichnung. Dabei bilden alle beschriebenen und/oder bildlich dargestellten Merkmale für sich oder in beliebiger Kombination den Gegenstand der Erfindung, auch unabhängig von ihrer Zusammenfassung in einzelnen Ansprüchen oder deren Rückbeziehung.

Die einzige Figur zeigt schematisch eine zur Durchführung des erfindungsgemäßen Verfahrens geeignete Anlage.

Die in der Fig. dargestellte Anlage umfasst einen ersten Reaktor 1 und einen zweiten Reaktor 2, welche jeweils einen Katalysator auf Basis von formselektivem Zeolith, vorzugsweise ein Alumosilikat-Zeolith vom Pentasiltyp und besonders bevorzugt ZSM-5, enthalten. Während der erste Reaktor 1 vorzugsweise als Schachtreaktor, Röhrenreaktor, stationärer Wirbelschichtreaktor oder zirkulierender Wirbelschichtreaktor ausgestaltet ist, ist der Katalysator in dem zweiten Reaktor 2 vorzugsweise als Festbett angeordnet. Des weiteren umfasst die Anlage eine als Kühler ausgestaltete erste Trenneinrichtung 3 sowie eine zweite Trenneinrichtung 4.

Beim Betrieb der Anlage wird über die Methanolzufuhrleitung 5 herangeführtes Methanol in einem Wärmeaustauscher (nicht dargestellt) auf eine Temperatur von vorzugsweise 200 bis 350°C erhitzt und dadurch verdampft, bevor der Methanoldampf in dem Vorreaktor 6 an einem geeigneten Dehydratisierungskatalysator, bspw. Aluminiumoxid, zumindest teilweise zu Dimethylether und Wasser umgesetzt wird. Dem über Leitung 7 aus dem Vorreaktor abgezogenen Methanol-/Dimethylethergemisch wird über die Gasleitung 8 Wasserdampf zugeführt und das so erhaltene Gemisch in den ersten Reaktor 1 geleitet. Vorzugsweise beträgt die Eintrittstemperatur des Eduktgemischs in den ersten Reaktor 1 zwischen 350 und 500°C, das Gewichtsverhältnis Wasser zu Methanoläquivalent in dem Eduktgemisch zwischen 0,25:1 und 6:1 sowie der Druck in dem ersten Reaktor 1 zwischen 0,5 und 5,0 bara. Im Katalysatorbereich des ersten Reaktors liegen die Temperaturen bevorzugt zwischen 300 und 600°C.

Alternativ zu der in der Fig. dargestellten einstufigen Ausgestaltung des ersten Reaktors 1 kann dieser auch aus zwei oder mehr in Serie geschalteten Reaktionsstufen bestehen, wobei es sich dabei sowohl um separat ausgeführte Reaktoren als auch um übereinander angeordnete Katalysatorschüttungen in einem Reaktor handeln kann. In diesem Fall wird das Produkt aus dem Vorreaktor 6 auf die einzelnen Stufen verteilt, während alle andere Eintrittsströme komplett in die erste Reaktionsstufe eingeleitet werden. Ferner ist es auch möglich, anstelle eines Wasserdampf-/ Methanol-/Dimethylethergemischs in dem ersten Reaktor 1 ausschließlich Methanol oder Dimethylether in Kombination mit Wasserdampf als Edukt einzusetzen.

Über Leitung 9 wird das in dem ersten Reaktor 1 gebildete und vornehmlich aus C₂-C₄-Olefinen, C₅₊-Benzinkohlenwasserstoffen und Wasserdampf bestehende Reaktionsgemisch aus dem ersten Reaktor 1 abgezogen und der ersten Trenneinrichtung 3 zugeführt, in dem das Reaktionsgemisch auf eine Temperatur zwischen 10 und 80°C abgekühlt wird, so dass ein wasserreiches Kondensat, eine flüssige organische Phase, welche reich an C₅₊-Benzinkohlenwasserstoffen ist, sowie eine im Wesentlichen aus C₂-C₄-Olefinen bestehende Gasphase erhalten wird.

Aus der ersten Trenneinrichtung 3 wird die C₂-C₄-olefinenreiche Fraktion über Leitung 10 einer dritten Trenneinrichtung 11 zugeführt, in der sie in einen C₄-C₅-Olefin- und einen C₃₋-Olefinstrom aufgetrennt wird. Während der erstgenannte Olefinstrom über die Rückführleitung 12 und die Methanol-/Dimethyletherabzugsleitung 7 in den ersten Reaktor 1 zurückgeführt wird, wird aus dem C₃₋-Olefinstrom über eine weitere Trenneinrichtung (nicht dargestellt) Propylen und Propan abgetrennt und wird der propylenfreie C₂₋-Reststrom über Leitung 15 in den ersten Reaktor zurückgeführt. Die weitere Aufreinigung des aus dem C₃₋-Olefinstroms abgetrennten Propylens kann mit aus dem Stand der Technik bekannten Verfahren erfolgen.

Die in der ersten Trenneinrichtung 3 gewonnene, im Wesentlichen aus C₅₋-Benzinkohlenwasserstoffen bestehende organische Phase wird über die Leitung 13 in die zweite, als Destillationskolonne ausgebildete Trenneinrichtung 4 geleitet, in der diese Phase in einen C₅-C₆-Kohlenwasserstoff- und einen C₇₊-Kohlenwasserstoffstrom aufgetrennt wird. Der erstgenannte Kohlenwasserstoffstrom wird über die Rückführleitung 12' und die Methanol-/Dimethyletherabzugsleitung 7 in den ersten Reaktor 1 zurückgeführt, wohingegen der C₇₊-Kohlenwasserstoffstrom über die Leitung 13', der via Leitung 14 aus der ersten Trenneinrichtung 3 als flüssiges Kondensat abgezogenes Wasser zugeführt wird, zunächst durch einen Erhitzer (nicht dargestellt), in dem das Gemisch aus C₇₊-Kohlenwasserstoffen und Wasser auf eine Temperatur von vorzugsweise 380 bis 600°C erwärmt wird, geführt und anschließend in den zweiten Reaktor 2 geleitet wird, in dem die vorgenannte Mischung an einem körnigen Zeolith-Katalysator zu einem C₂-C₄-Olefine umfassenden Produktgemisch umgesetzt wird. Über die Rückfuhrleitung 12" wird dieses Produktgemisch aus dem zweiten Reaktor 2 abgezogen und in die erste Trenneinrichtung 3 zurückgeführt.

Um eine zu hohe Konzentration inerter Stoffe im Prozesskreislauf zu vermeiden, wird ein kleiner Anteil der C₂-C₄-Olefine, der C₅-C₆-Kohlenwasserstoffe und der C₇₊-Kohlenwasserstoffe kontinuierlich aus dem Prozesskreislauf ausgeschleust (nicht dargestellt).

Im folgenden wird die Erfindung anhand eines Beispiels erläutert.

### Beispiel 1

In einer der Fig. entsprechenden Anlage wurde dem ersten Reaktor 1 über die Leitungen 5, 7, 8 in dem ersten Reaktor 1, 2,23 kg/h einer Mischung aus 8,7 Gew.-% Methanoldampf, 32,9 Gew.-% Dimethyletherdampf und 58,3 Gew.-% Wasserdampf, über die Leitungen 12, 7 0,75 kg/h des aus der dritten Trenneinrichtung abgezogenen C₄-C₅-Olefinstroms, über die Leitungen 15, 7 0,13 kg/h Ethylen sowie über die Leitungen 12', 7 0,18 kg/h des aus der zweiten Trenneinrichtung abgezogenen C₅-C₆-Kohlenwasserstoffstroms zugeführt, wobei die Eintrittstemperatur der Mischung in den Reaktor 1 etwa 459°C und der Druck am Eintritt von Reaktor 1 etwa 2,15 bara betrug.

Aus dem Reaktor 1 wurde über Leitung 9 das Reaktionsgemisch abgezogen und in die als Kühler ausgebildete erste Trenneinrichtung 3 geleitet, in der das Reaktionsgemisch auf 24°C abgekühlt wurde. Aus dem Sumpf der Trenneinrichtung 3 wurden, bezogen auf ein Kilogramm Katalysator in dem ersten Reaktor 1, 0,33 kg/h einer an C₅₊-Benzinkohlenwasserstoffen reichen Fraktion abgezogen, die in der zweiten Trenneinrichtung 4 in einen C₅-C₆-Kohlenwasserstoffe enthaltenden und einen C₇₊-Kohlenwasserstoffen enthaltenden Produktstrom aufgetrennt wurden, wobei der C₇₋-Kohlenwasserstoffe enthaltende Produktstrom die nachfolgende Zusammensetzung:
- 18,5 Gew.-% C₇₊-Paraffine,
- 42,4 Gew.-% Aromaten,
- 18,7 Gew.-% Naphthene,
- 19,2 Gew.-% C₇₊-Olefine sowie
- weniger als 0,3 Gew.-% C₆₋-Kohlenwasserstoffe
und der C₅-C₆-Kohlenwasserstoffe enthaltende Produktstrom die folgende Zusammensetzung aufwies:
- 32,6 Gew.-% C₇₋-Paraffine,
- 0,2 Gew.-% Aromaten
- 14,3 Gew.-% Naphthene sowie
- 52,9 Gew.-% C7₋-Olefine.

Während der C₅-C₆-Kohlenwasserstoffe enthaltende Produktstrom über die Leitungen 12', 7 in den ersten Reaktor 1 zurückgeführt wurde, wurde der C₇₊-Kohlenwasserstoffe enthaltende Produktstrom mit einem Massenstrom, bezogen auf ein Kilogramm Katalysator in dem zweiten Reaktor 2, von 3 kg/h mit 1,5 kg/h Wasserdampf vermischt, die so erhaltene Mischung erhitzt und mit einer Eintrittstemperatur von etwa 480°C in den zweiten Reaktor 2, der bei einem Druck von 1,3 bara betrieben wurde, geleitet. Das aus dem Reaktor 2 abgezogene Produkt wurde über die Leitung 12" mit einem Massenstrom von 0,095 kg/h in die erste Trenneinrichtung 3 zurückgeführt.

Zudem wurde aus der ersten Trenneinrichtung ein C₂-C₄-olefinreiches Gemisch abgezogen und über Leitung 10 einer dritten Trenneinrichtung 11 zugeführt, in der dieser Strom in einen C₄-C₅-Olefin- und einen C₃₋-Olefinstrom aufgetrennt wurde.

Aus dem C₃₋-Olefinstrom wurde Propylen in einer Ausbeute von 69,3% gewonnen.

### Bezugszeichenliste:

- 1: erster Zeolith-Reaktor
- 2: zweiter Zeolith-Reaktor
- 3: erste Trenneinrichtung
- 4: zweite Trenneinrichtung
- 5: Methanolzufuhrleitung
- 6: Vorreaktor
- 7: Methanol-/Dimethyletherabzugsleitung
- 8: Wasserdampfzufuhrleitung
- 9: Reaktionsgemischabzugsleitung
- 10: Gasleitung
- 11: dritte Trenneinrichtung
- 12, 12', 12": Rückführleitung
- 13, 13': Flüssigkeitsleitung
- 14: Wasserzufuhrleitung
- 15: Ethylenrückführleitung

## Patentansprüche

1. Verfahren zur Herstellung von C₂-C₄-Olefinen, insbesondere von Propylen, aus einem Wasserdampf sowie Methanol- und/oder Dimethyletherdampf enthaltenden Eduktgemisch, bei dem das Eduktgemisch in wenigstens einem ersten Reaktor (1) an einem körnigen, formselektiven Zeolith-Katalysator zu einem niedermolekulare Olefine und Benzinkohlenwasserstoffe umfassenden Reaktionsgemisch umgesetzt wird, welches in einer ersten Trenneinrichtung (3) in ein C₂-C₄-Olefinreiches Gemisch, ein an C₅₊-Benzinkohlenwasserstoffen reiches Gemisch und eine wässrige Phase aufgetrennt wird, wobei das benzinkohlenwasserstoffreiche Gemisch mit einem inerten Medium vermischt, die so erhaltene Mischung in wenigstens einem zweiten Reaktor (2) an körnigem Zeolith-Katalysator zu einem C₂-C₄-Olefine umfassenden Produktgemisch umgesetzt und dieses Produktgemisch zu der ersten Trenneinrichtung (3) zurückgeführt wird, **dadurch gekennzeichnet, dass** das an C₅₊-Benzinkohlenwasserstoffen reiche Gemisch vor der Zuführung zu dem zweiten Reaktor (2) in einer zweiten Trenneinrichtung (4) in einen C₅-C₆-Kohlenwasserstoffe enthaltenden und einen C₇₊-Kohlenwasserstoffe enthaltenden Produktstrom aufgetrennt wird und nur der C₇₊-Kohlenwasserstoffe enthaltende Produktstrom dem zweiten Reaktor (2) zugeführt wird, wohingegen der C₅-C₆-Kohlenwasserstoffe enthaltende Produktstrom zusammen mit dem Eduktgemisch dem wenigstens einen ersten Reaktor (1) zugeführt wird.

2. Verfahren nach Anspruch 1, **dadurch gekennzeichnet, dass** der in der zweiten Trenneinrichtung (4) abgetrennte, C₇₊-Kohlenwasserstoffe enthaltende Produktstrom 10 bis 30 Gew.-% C₇₊-Paraffine, 40 bis 70 Gew.-% Aromaten, 5 bis 20 Gew.-% Naphthene, 5 bis 25 Gew.-% C₇₊-Olefine sowie weniger als 20 Gew.-% C₆₋-Kohlenwasserstoffe, und bevorzugt 15 bis 25 Gew.-% C₇₊-Paraffine, 40 bis 50 Gew.-% Aromaten, 15 bis 20 Gew.-% Naphthene, 15 bis 25 Gew.-% C₇₊-Olefine sowie weniger als 5 Gew.-% C₆₋-Kohlenwasserstoffe enthält.

3. Verfahren nach Anspruch 1 oder 2, **dadurch gekennzeichnet, dass** der in der zweiten Trenneinrichtung (4) abgetrennte, C₅-C₆-Kohlenwasserstoffe enthaltende Produktstrom weniger als 5 Gew.-% Aromaten, bevorzugt weniger als 2,5 Gew.-% Aromaten und besonders bevorzugt weniger als 1 Gew.-% Aromaten enthält.

4. Verfahren nach einem der vorhergehenden Ansprüche, **dadurch gekennzeichnet, dass** die zweite Trenneinrichtung (4) als Destillationskolonne ausgebildet ist.

5. Verfahren nach einem der vorhergehenden Ansprüche, **dadurch gekennzeichnet, dass** das C₇₊-benzinkohlenwasserstoffreiche Gemisch vor der Zuführung in den zweiten Reaktor (2) mit Wasser, Stickstoff und/oder Butan als inertem Medium vermischt wird.

6. Verfahren nach einem der vorhergehenden Ansprüche, **dadurch gekennzeichnet, dass** der wenigstens eine erste Reaktor (1) als Schachtreaktor, Röhrenreaktor, stationärer Wirbelschichtreaktor oder zirkulierender Wirbelschichtreaktor ausgebildet ist.

7. Verfahren nach einem der vorhergehenden Ansprüche, **dadurch gekennzeichnet, dass** zwei oder mehr hintereinander geschaltete erste Reaktoren (1) eingesetzt werden oder ein erster Reaktor (1) umfassend wenigstens zwei hintereinander angeordnete Katalysatorstufen eingesetzt wird.

8. Verfahren nach einem der vorhergehenden Ansprüche, **dadurch gekennzeichnet, dass** der Katalysator in dem ersten und/oder zweiten Reaktor (1, 2) ein Alumosilikat-Zeolith vom Pentasiltyp, bevorzugt ZSM-5, ist.

9. Verfahren nach einem der vorhergehenden Ansprüche, **dadurch gekennzeichnet, dass** die Umsetzung in dem wenigstens einen ersten Reaktor (1) und/oder in dem wenigstens einen zweiten Reaktor (2) adiabatisch durchgeführt wird.

10. Verfahren nach einem der vorhergehenden Ansprüche, **dadurch gekennzeichnet, dass** das Gewichtsverhältnis Wasser zu Methanoläquivalent, bezogen auf alle Methanol-Eintrittsströme, des dem ersten Reaktor (1) zugeführten Eduktgemischs auf 0,25:1 bis 6:1 eingestellt wird und das Eduktgemisch in dem ersten Reaktor (1) bei einer Temperatur von 300 bis 600°C und/oder bei einem Druck von 0,5 und 5 bara umgesetzt wird.

11. Verfahren nach einem der vorhergehenden Ansprüche, **dadurch gekennzeichnet, dass** die erste Trenneinrichtung (3) eine Kühlvorrichtung ist, in der das Reaktionsgemisch auf eine Temperatur von 10 bis 80°C abgekühlt wird.

12. Verfahren nach einem der vorhergehenden Ansprüche, **dadurch gekennzeichnet, dass** das in der ersten Trenneinrichtung (3) erhaltene C₂-C₄-Olefinreiche Gemisch einer dritten Trenneinrichtung (11) zugeführt wird, in der das Gemisch in einen C₄-C₅-Olefin- und einen C₃₋-Olefinstrom aufgetrennt wird.

13. Verfahren nach Anspruch 12, **dadurch gekennzeichnet, dass** der C₄-C₅-Olefinstrom in den ersten Reaktor (1) zurückgeführt wird.

14. Verfahren nach Anspruch 12 oder 13, **dadurch gekennzeichnet, dass** aus dem C₃₋-Olefinstrom Propylen abgetrennt wird und ggf. die verbleibenden Olefine in den ersten Reaktor (1) zurückgeführt werden.

15. Verfahren nach einem der vorhergehenden Ansprüche, **dadurch gekennzeichnet, dass** das Verhältnis Wasser zu Kohlenwasserstoff des dem zweiten Reaktor (2) zugeführten Produktstroms auf 0,1:1 bis 3:1, bevorzugt auf mindestens 1:1, eingestellt wird und der Produktstrom in dem zweiten Reaktor (2) bei einer Temperatur von 380 bis 700°C, bevorzugt 400 bis 600°C, und/oder bei einem Druck von 0,2 und 5 bara, bevorzugt 1,0 bis 2,5 bara, umgesetzt wird.

16. Anlage zur Herstellung von C₂-C₄-Olefinen, insbesondere von Propylen, aus einem Wasserdampf sowie Methanol- und/oder Dimethyletherdampf enthaltenden Eduktgemisch, insbesondere geeignet zur Durchführung eines Verfahrens nach einem der Ansprüche 1 bis 15, umfassend wenigstens einen ersten Reaktor (1) und wenigstens einen zweiten Reaktor (2), welche jeweils einen körnigen, formselektiven Zeolith-Katalysator enthalten, sowie eine erste Trenneinrichtung (3) zur Auftrennung des in dem ersten Reaktor (1) erhaltenen Reaktionsgemischs, **gekennzeichnet durch** eine zweite Trenneinrichtung (4), welche daran angepasst ist, ein an C₅₊-Benzinkohlenwasserstoffen reiches Gemisch vor der Zuführung in den zweiten Reaktor (2) in einen C₅-C₆-Kohlenwasserstoffe enthaltenden und einen C₇₋-Kohlenwasserstoffen enthaltenden Produktstrom aufzutrennen, und **durch** eine Rückfuhrleitung (12"), die von dem zweiten Reaktor (2) zu der ersten Trenneinrichtung (3) führt.

17. Anlage nach Anspruch 16, **gekennzeichnet durch** eine der ersten Trenneinrichtung (3) nachgeschaltete dritte Trenneinrichtung (11), welche zumindest eine Destillationskolonne umfasst.

18. Anlage nach Anspruch 17, **gekennzeichnet durch** eine von der dritten Trenneinrichtung (11) zu dem ersten Reaktor (1) führende Rückfuhrleitung (12).

## Claims

1. A process for producing C₂-C₄ olefins, in particular propylene, from an educt mixture containing steam as well as methanol vapour and/or dimethyl ether vapour, in which the educt mixture is reacted in at least one first reactor (1) on a granular, form-selective zeolite catalyst to obtain a reaction mixture comprising low-molecular olefins and gasoline hydrocarbons, which in a first separating device (3) is separated into a mixture rich in C₂-C₄ olefins, a mixture rich in C₅₊ gasoline hydrocarbons, and an aqueous phase, wherein the mixture rich in gasoline hydrocarbons is mixed with an inert medium, the mixture thus obtained is reacted in at least one second reactor (2) on a granular zeolite catalyst to obtain a product mixture comprising C₂-C₄ olefins, and this product mixture is recirculated to the first separating device (3), **characterized in that** before being supplied to the second reactor (2), the mixture rich in C₅₊ gasoline hydrocarbons is separated in a second separating device (4) into a product stream containing C₅-C₆ hydrocarbons and a product stream containing C₇₊ hydrocarbons and only the product stream containing C₇₊ hydrocarbons is supplied to the second reactor (2), whereas the product stream containing C₅-C₆ hydrocarbons is supplied to the at least one first reactor (1) together with the educt mixture.

2. The process as claimed in claim 1, **characterized in that** the product stream containing C₇₊ hydrocarbons, which was separated in the second separating device (4), contains 10 to 30 wt-% of C₇₊ paraffins, 40 to 70 wt-% of aromatics, 5 to 20 wt-% of naphthenes, 5 to 25 wt-% of C₇₊ olefins as well as less than 20 wt-% of C₆₋ hydrocarbons, and preferably 15 to 25 wt-% of C₇₊ paraffins, 40 to 50 wt-% of aromatics, 15 to 20 wt-% of naphthenes, 15 to 25 wt-% of C₇₊ olefins and less than 5 wt-% of C₆₋ hydrocarbons.

3. The process as claimed in claim 1 or 2, **characterized in that** the product stream containing C₅-C₆ hydrocarbons, which was separated in the second separating device (4), contains less than 5 wt-% of aromatics, preferably less than 2.5 wt-% of aromatics and particularly preferably less than 1 wt-% of aromatics.

4. The process as claimed in any of the preceding claims, **characterized in that** the second separating device (4) constitutes a distillation column.

5. The process as claimed in any of the preceding claims, **characterized in that** before being supplied to the second reactor (2), the mixture rich in C₇₊ gasoline hydrocarbons is mixed with water, nitrogen and/or butane as inert medium.

6. The process as claimed in any of the preceding claims, **characterized in that** the at least one first reactor (1) constitutes a fixed bed reactor, tubular reactor, stationary fluidized-bed reactor or circulating fluidized-bed reactor.

7. The process as claimed in any of the preceding claims, **characterized in that** two or more sequentially operated first reactors (1) are used or a first reactor (1) comprising at least two sequentially operated catalyst stages is used.

8. The process as claimed in any of the preceding claims, **characterized in that** the catalyst in the first and/or second reactor (1, 2) is an alumosilicate-zeolite of the pentasile type, preferably ZSM-5.

9. The process as claimed in any of the preceding claims, **characterized in that** the reaction in the at least one first reactor (1) and/or in the at least one second reactor (2) is performed adiabatically.

10. The process as claimed in any of the preceding claims, **characterized in that** the weight ratio of water to methanol equivalent, based on all methanol inlet streams, of the educt mixture supplied to the first reactor (1) is adjusted to 0.25:1 to 6:1, and the educt mixture is reacted in the first reactor (1) at a temperature of 300 to 600°C and/or at a pressure of 0.5 to 5 bara.

11. The process as claimed in any of the preceding claims, **characterized in that** the first separating device (3) is a cooling device, in which the reaction mixture is cooled to a temperature of 10 to 80°C.

12. The process as claimed in any of the preceding claims, **characterized in that** the mixture rich in C₂-C₄ olefins, which was obtained in the first separating device (3), is supplied to a third separating device (11), in which the mixture is separated into a C₄-C₅ olefin stream and a C₃₋ olefin stream.

13. The process as claimed in claim 12, **characterized in that** the C₄-C₅ olefin stream is recirculated to the first reactor (1).

14. The process as claimed in claim 12 or 13, **characterized in that** propylene is separated from the C₃₋ olefin stream and the remaining olefins are recirculated to the first reactor (1).

15. The process as claimed in any of the preceding claims, **characterized in that** the water/hydrocarbon ratio of the product stream supplied to the second reactor (2) is adjusted to 0.1:1 to 3:1, preferably to at least 1:1, and the product stream in the second reactor (2) is reacted at a temperature of 380 to 700°C, preferably 400 to 600°C and/or at a pressure of 0.2 to 5 bara, preferably 1.0 to 2.5 bara.

16. A plant for producing C₂-C₄ olefins, in particular propylene, from an educt mixture containing steam as well as methanol vapour and/or dimethyl ether vapour, which is particularly useful for performing a process as claimed in any of claims 1 to 15, comprising at least one first reactor (1) and at least one second reactor (2), each of which contains a granular form-selective zeolite catalyst, as well as a first separating device (3) for separating the reaction mixture obtained in the first reactor (1), **characterized by** a second separating device (4) which is adapted to separate a mixture rich in C₅₊ gasoline hydrocarbons into a product stream containing C₅-C₆ hydrocarbons and a product stream containing C₇₊ hydrocarbons before supplying the same to the second reactor (2), and by a return line (12") which leads from the second reactor (2) to the first separating device (3).

17. The plant as claimed in claim 16, **characterized by** a third separating device (11) downstream of the first separating device (3), which comprises at least one distillation column.

18. The plant as claimed in claim 17, **characterized by** a return line (12) leading from the third separating device (11) to the first reactor (1).

## Revendications

1. Procédé de préparation d'oléfines de type C₂-C₄, notamment de propylène, à partir d'un mélange de produits de départ contenant de la vapeur d'eau ainsi que de la vapeur de méthanol et/ou d'éther diméthylique, en faisant réagir ledit mélange de produits de départ dans un premier réacteur (1) sur un catalyseur de zéolithe granulé à sélectivité de forme, pour obtenir un mélange réactionnel comprenant des oléfines à faible masse moléculaire et des hydrocarbures d'essence qui, dans un premier dispositif de séparation (3), est séparé en un mélange riche en oléfines de type C₂-C₄, en un mélange riche en hydrocarbures d'essence de type C₅₊ et en une phase aqueuse, ledit mélange riche en hydrocarbures d'essence étant mélangé avec un milieu inerte pour ainsi obtenir un mélange que l'on fait réagir, dans au moins un deuxième réacteur (2), sur un catalyseur de zéolithe granulé, pour obtenir un mélange de produits comprenant des oléfines de type C₂-C₄ et recycler ledit mélange de produits dans le premier dispositif de séparation (3), **caractérisé en ce que** ledit mélange riche en hydrocarbures d'essence de type C₅₊ est séparé, avant d'être introduit dans le deuxième réacteur (2), dans un deuxième dispositif de séparation (4), en un flux de produits contenant des hydrocarbures de type C₅-C₆ et en un autre contenant des hydrocarbures de type C₇₊, et seul le flux de produits contenant des hydrocarbures de type C₇₊ est introduit dans le deuxième réacteur (2) alors que le flux de produits contenant des hydrocarbures de type C₅-C₆ est introduit, ensemble avec ledit mélange de produits de départ, dans l'au moins un premier réacteur (1).

2. Procédé selon la revendication 1, **caractérisé en ce que** le flux de produits contenant des hydrocarbures de type C₇₊ qui est séparé dans le deuxième dispositif de séparation (4) contient 10 à 30 % en poids de paraffines de type C₇₊, 40 à 70 % des composés aromatiques, 5 à 20 % en poids de naphtènes, 5 à 25 % en poids d'oléfines de type C₇₊ ainsi que moins de 20 % en poids d'hydrocarbures de type C₆₋ et, de préférence, 15 à 25 % en poids de paraffines de type C₇₊, 40 à 50 % des composés aromatiques, 15 à 20 % en poids de naphtènes, 15 à 25 % en poids d'oléfines de type C₇₊ ainsi que moins de 5 % en poids d'hydrocarbures de type C₆₋.

3. Procédé selon la revendication 1 ou 2, **caractérisé en ce que** le flux de produits contenant des hydrocarbures de type C₅-C₆ séparé dans le deuxième dispositif de séparation (4) contient moins de 5 % en poids de composés aromatiques, de préférence moins de 2,5 % en poids de composés aromatiques et, d'une manière particulièrement préférée, moins de 1 % de composés aromatiques.

4. Procédé selon l'une des revendications précédentes, **caractérisé en ce que** le deuxième dispositif de séparation (4) est réalisé sous forme d'une colonne de distillation.

5. Procédé selon l'une des revendications précédentes, **caractérisé en ce que** ledit mélange riche en hydrocarbures d'essence de type C₇₊ est mélangé avec de l'eau, de l'azote et/ou du butane en tant que milieu inerte, avant d'être introduit dans le deuxième réacteur (2).

6. Procédé selon l'une des revendications précédentes, **caractérisé en ce que** l'au moins un premier réacteur (1) est réalisé sous forme d'un réacteur à cuve verticale, d'un réacteur tubulaire, d'un réacteur à lit fluidisé stationnaire ou d'un réacteur à lit fluidisé avec circulation.

7. Procédé selon l'une des revendications précédentes, **caractérisé en ce que** l'on met en oeuvre au moins deux premiers réacteurs (1) ou l'on met en oeuvre un premier réacteur (1) comprenant au moins deux étages de catalyseur disposés en série.

8. Procédé selon l'une des revendications précédentes, **caractérisé en ce que** le catalyseur dans le premier et/ou deuxième réacteur (1, 2) est une zéolithe alumosilicate de type pentasil, de préférence ZSM-5.

9. Procédé selon l'une des revendications précédentes, **caractérisé en ce que** la réaction dans l'au moins un premier réacteur (1) et/ou dans l'au moins un deuxième réacteur (2) est réalisée de façon adiabatique.

10. Procédé selon l'une des revendications précédentes, **caractérisé en ce que** le rapport de poids entre l'eau et l'équivalent méthanol, par rapport à l'ensemble des flux entrants de méthanol du mélange de produits de départ introduit dans le premier réacteur (1), est réglé tel qu'il et de 0,25:1 à 6:1 et que l'on fait réagir, dans le premier réacteur (1), ledit mélange de produits de départ à une température de 300 à 600 °C et/ou à une pression de 0,5 à 5 bar absolue.

11. Procédé selon l'une des revendications précédentes, **caractérisé en ce que** le premier dispositif de séparation (3) est un dispositif de refroidissement dans lequel le mélange réactionnel est refroidi à une température de 10 à 80 °C.

12. Procédé selon l'une des revendications précédentes, **caractérisé en ce que** le mélange riche en oléfines de type C₂-C₄ obtenu dans le premier dispositif de séparation (3) est introduit dans un troisième dispositif de séparation (11) dans lequel ledit mélange est séparé en un flux d'oléfines de type C₄-C₅ et un autre d'oléfines de type C₃₋.

13. Procédé selon la revendication 12, **caractérisé en ce que** ledit flux d'oléfines de type de type C₄-C₅ est recyclé dans le premier réacteur (1).

14. Procédé selon la revendication 12 ou 13, **caractérisé en ce que** le propylène est séparé du flux d'oléfines de type C₃₋ et les oléfines restantes sont, le cas échéant, recyclés dans le premier réacteur (1).

15. Procédé selon l'une des revendications précédentes, **caractérisé en ce que** le rapport entre l'eau et les hydrocarbures du flux de produits introduit dans le deuxième réacteur (2) est réglé tel qu'il est de 0,1:1 à 3:1, de préférence d'au moins 1:1, et dans le deuxième réacteur (2), on fait réagir le flux de produits à une température de 380 à 700 °C, de préférence de 400 à 600 °C et/ou à une pression de 0,2 à 5 bar absolue, de préférence de 1,0 à 2,5 bar absolue.

16. Installation destinée à la préparation d'oléfines de type C₂-C₄, notamment de propylène, à partir d'un mélange de produits de départ contenant de la vapeur d'eau ainsi que de la vapeur de méthanol et/ou d'éther diméthylique, qui est particulièrement adaptée à la mise en oeuvre d'un procédé selon l'une des revendications 1 à 15, comprenant au moins un premier réacteur (1) et au moins un deuxième réacteur (2) contenant chacun un catalyseur de zéolithe granulé à sélectivité de forme ainsi qu'un premier dispositif de séparation (3) destiné à la séparation du mélange réactionnel obtenu dans le premier réacteur (1), **caractérisée par** un deuxième dispositif de séparation (4), adapté pour séparer un mélange riche en hydrocarbures d'essence de type C₅₊, avant qu'il ne soit introduit dans le deuxième réacteur (2), en un flux de produits contenant des hydrocarbures de type C₅-C₆ et en un autre contenant des hydrocarbures de type C₇₊, et par une ligne de recyclage (12") menant du deuxième réacteur (2) au premier dispositif de séparation (3).

17. Installation selon la revendication 16, **caractérisée par** un troisième dispositif de séparation (11) qui est situé en aval du premier dispositif de séparation (3) et qui comprend au moins une colonne de distillation.

18. Installation selon la revendication 17, **caractérisée par** une ligne de recyclage (12) menant du troisième dispositif de séparation (11) au premier réacteur (1).
